(19) Europäisches Patentamt
European Patent Office
Office européen des brevets

(11)  **EP 0 815 263 B1**

(12)  **EUROPEAN PATENT SPECIFICATION**

(45) Date of publication and mention
of the grant of the patent:
**21.04.2004 Bulletin 2004/17**

(51) Int Cl.[7]: **C12Q 1/68**

(21) Application number: **96944531.1**

(22) Date of filing: **20.12.1996**

(86) International application number:
**PCT/US1996/020627**

(87) International publication number:
**WO 1997/023651 (03.07.1997 Gazette 1997/29)**

(54) **METHODS FOR THE DETECTION OF CLONAL POPULATIONS OF TRANSFORMED CELLS IN A GENOMICALLY HETEROGENEOUS CELLULAR SAMPLE**

VERFAHREN ZUR ERKENNUNG VON KLONALEN POPULATIONEN VON TRANSFORMIERTEN ZELLEN IN EINER GENOMISCH HETEROGENEN ZELLULÄREN PROBE

PROCEDES RELATIFS A LA DETECTION DES POPULATIONS CLONALES DE CELLULES TRANSFORMEES DANS UN ECHANTILLON CELLULAIRE A GENOMES HETEROGENES

(84) Designated Contracting States:
**AT BE CH DE DK ES FI FR GB GR IE IT LI LU MC NL PT SE**

(30) Priority: **22.12.1995 US 9137 P**
**14.08.1996 US 700583**

(43) Date of publication of application:
**07.01.1998 Bulletin 1998/02**

(73) Proprietor: **EXACT Sciences Corporation**
**Marlborough, Massachusetts 01752 (US)**

(72) Inventors:
• **LAPIDUS, Stanley, N.**
**Bedford, NH 03110 (US)**
• **SHUBER, Anthony, P.**
**Milford, MA 01757 (US)**
• **ULMER, Kevin, M.**
**Cohasset, MA 02025 (US)**

(74) Representative: **Kirkham, Nicholas Andrew et al**
**Graham Watt & Co.**
**St. Botolph's House**
**7-9 St. Botolph's Road**
**Sevenoaks Kent TN13 3AJ (GB)**

(56) References cited:
EP-A- 0 664 339          WO-A-93/18186
WO-A-94/09161          WO-A-94/11383
WO-A-95/09929          US-A- 5 302 509

• SCIENCE, vol. 256, 1992, LANCASTER, PA US, pages 102-105, XP000605488 SIDRANSKI ET AL.: "Identification of ras oncogene mutations in the stool of patients with curable colorectal tumors." cited in the application
• PROCEEDINGS OF THE NATIONAL ACADEMY OF SCIENCES OF USA, vol. 92, January 1995, pages 83-85, XP000606043 JONSSON J J ET AL: "FROM MUTATION MAPPING TO PHENOTYPE CLONING"
• CANCER RESEARCH, vol. 54, no. 17, 1 September 1994, pages 4598-4602, XP000576043 WATSON M A ET AL: "ISOLATION OF DIFFERENTIALLY EXPRESSED SEQUENCE TAGS FROM HUMAN BREAST CANCER"
• SCIENCE, vol. 259, 12 February 1993, page 942/943 XP000601303 MYERS R M: "THE PLUSES OF SUBTRACTION"

SEQUENCE LISTING

(1) GENERAL INFORMATION:

(i) APPLICANT:

(A) NAME: EXACT LABORATORIES, INC.
(B) STREET: 12 OLD EVERGREEN ROAD
(C) CITY: BEDFORD
(D) STATE: NEW HAMPSHIRE
(E) COUNTRY: USA
(F) POSTAL CODE (ZIP): 03110
(G) TELEPHONE:
(H) TELEFAX:
(I) TELEX:

(ii) TITLE OF INVENTION: METHODS FOR THE DETECTION OF CLONAL POPULATIONS OF TRANSFORMED CELLS IN A GENOMICALLY HETEROGENEOUS CELLULAR SAMPLE

(iii) NUMBER OF SEQUENCES: 8

(iv) CORRESPONDENCE ADDRESS:

(A) ADDRESSEE: PATENT ADMINISTRATOR, TESTA, HURWITZ & THIBEAULT, LLP
(B) STREET: 125 HIGH STREET
(C) CITY: BOSTON
(D) STATE: MA
(E) COUNTRY: USA
(F) ZIP: 02110

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:

(A) APPLICATION NUMBER:
(B) FILING DATE:
(C) CLASSIFICATION:

(viii) ATTORNEY/AGENT INFORMATION:

(A) NAME: MEYERS, THOMAS C
(B) REGISTRATION NUMBER: 36,989
(C) REFERENCE/DOCKET NUMBER: EXT-001PC

(ix) TELECOMMUNICATION INFORMATION:

(A) TELEPHONE: (617) 248-7000
(B) TELEFAX: (617) 248-7100

(2) INFORMATION FOR SEQ ID NO:1:

(i) SEQUENCE CHARACTERISTICS:

    (A) LENGTH: 9 base pairs
    (B) TYPE: nucleic acid
    (C) STRANDEDNESS: single
    (D) TOPOLOGY: linear

(ix) FEATURE:

    (A) NAME/KEY: misc_feature
    (B) LOCATION: 1..9
    (D) OTHER INFORMATION: /note= "M1"

(xi) SEQUENCE DESCRIPTION: SEQ ID NO:1:

**GGCATCGCA**                **9**

(2) INFORMATION FOR SEQ ID NO:2:

(i) SEQUENCE CHARACTERISTICS:

    (A) LENGTH: 19 base pairs
    (B) TYPE: nucleic acid
    (C) STRANDEDNESS: single
    (D) TOPOLOGY: linear

(ix) FEATURE:

    (A) NAME/KEY: misc_feature
    (B) LOCATION: 1..19
    (D) OTHER INFORMATION: /note= "M2"

(xi) SEQUENCE DESCRIPTION: SEQ ID NO:2:

**ATCGGCTTAC TGCGATGCC**        **19**

(2) INFORMATION FOR SEQ ID NO:3:

(i) SEQUENCE CHARACTERISTICS:

    (A) LENGTH: 19 base pairs
    (B) TYPE: nucleic acid
    (C) STRANDEDNESS: single
    (D) TOPOLOGY: linear

(ix) FEATURE:

    (A) NAME/KEY: misc feature
    (B) LOCATION: 1..19
    (D) OTHER INFORMATION: /note= "M3"

(xi) SEQUENCE DESCRIPTION: SEQ ID NO:3:

**GGCATCGCAG TAAGCCGAT**        **19**

(2) INFORMATION FOR SEQ ID NO:4:

    (i) SEQUENCE CHARACTERISTICS:

        (A) LENGTH: 9 base pairs
        (B) TYPE: nucleic acid
        (C) STRANDEDNESS: single
        (D) TOPOLOGY: linear

    (ix) FEATURE:

        (A) NAME/KEY: misc feature
        (B) LOCATION: 1..9
        (D) OTHER INFORMATION: /note= "M4"

    (xi) SEQUENCE DESCRIPTION: SEQ ID NO:4:


**ATCGGCTTA**                                                                              9


(2) INFORMATION FOR SEQ ID NO:5:

    (i) SEQUENCE CHARACTERISTICS:

        (A) LENGTH: 9 base pairs
        (B) TYPE: nucleic acid
        (C) STRANDEDNESS: single
        (D) TOPOLOGY: linear

    (ix) FEATURE:

        (A) NAME/KEY: misc_feature
        (B) LOCATION: 1..9
        (D) OTHER INFORMATION: /note= "F1"

    (xi) SEQUENCE DESCRIPTION: SEQ ID NO:5:


**GGCATCGCA**                                                                              9


(2) INFORMATION FOR SEQ ID NO:6:

    (i) SEQUENCE CHARACTERISTICS:

        (A) LENGTH: 19 base pairs
        (B) TYPE: nucleic acid
        (C) STRANDEDNESS: single
        (D) TOPOLOGY: linear

    (ix) FEATURE:

        (A) NAME/KEY: misc_feature
        (B) LOCATION: 1..19
        (D) OTHER INFORMATION: /note= "F2"

    (xi) SEQUENCE DESCRIPTION: SEQ ID NO:6:

```
ATCGGCTTAT TGCGATGCC                                                    19
```

(2) INFORMATION FOR SEQ ID NO:7:

    (i) SEQUENCE CHARACTERISTICS:

        (A) LENGTH: 19 base pairs
        (B) TYPE: nucleic acid
        (C) STRANDEDNESS: single
        (D) TOPOLOGY: linear

    (ix) FEATURE:

        (A) NAME/KEY: misc_feature
        (B) LOCATION: 1..19
        (D) OTHER INFORMATION: /note= "F3"

    (xi) SEQUENCE DESCRIPTION: SEQ ID NO:7:

```
GGCATCGCAA TAAGCCGAT                .                                   19
```

(2) INFORMATION FOR SEQ ID NO:8:

    (i) SEQUENCE CHARACTERISTICS:

        (A) LENGTH: 9 base pairs
        (B) TYPE: nucleic acid
        (C) STRANDEDNESS: single
        (D) TOPOLOGY: linear

    (ix) FEATURE:

        (A) NAME/KEY: misc_feature
        (B) LOCATION: 1..9
        (D) OTHER INFORMATION: /note= "F4"

    (xi) SEQUENCE DESCRIPTION: SEQ ID NO:8:

```
ATCGGCTTA                                                                9
```

**Description**

**FIELD OF THE INVENTION**

This invention relates to methods useful for disease diagnosis by detecting the presence of genetic mutations, including deletions, in cellular samples containing a small amount of mutated genetic material dispersed within a major amount of diagnostically-irrelevant (normal) genetic material. Methods of the invention are especially useful in the detection of genetic mutations characteristic of cancer.

**BACKGROUND OF THE INVENTION**

Cancer is a disease characterized by genomic instability. Generally, genomic instability defines a broad class of disruptions in genomic nucleotide sequences. Such disruptions include the loss of heterozygosity (usually characterized by massive loss of chromosomal DNA), microsatellite instability (usually indicative of defects in DNA repair mechanisms), and mutations (which include insertions, deletions, substitutions, duplications, rearrangements, or modifications). Numerous genomic instabilities have been associated with cancer. For example, mutations in a number of oncogenes and tumor suppressor genes have been implicated in tumorigenesis. Duffy, *Clin. Chem., 41*: 1410-1413 (1993). In addition, the loss of heterozygosity at the P53 tumor suppressor locus has been correlated with various types of cancer. Ridanpaa, *et al.*, *Path. Res. Pract, 191*: 399-402 (1995). The loss or other mutation of the apc and dcc tumor suppressor genes has also been associated with tumor development. Blum, *Europ. J. Cancer, 31A:* 1369-372 (1995). Finally, tumorigenesis has also been correlated with microsatellite instability.

Genetic changes characteristic of genomic instability theoretically can serve as markers for the early stages of, for example, colon cancer, and can be detected in DNA isolated from biopsied colonic epithelium and in some cases from transformed cells shed into fecal material. Sidransky, *et al., Science, 256*: 102-105 (1992).

Detection methods proposed in the art are time-consuming and expensive. Duffy, *supra.* Moreover, methods according to the art cannot be used to identify a loss of heterozygosity or microsatellite instability in small subpopulation of cells when the cells exist in a heterogeneous (i.e., clonally impure) sample. For example, in U.S. Patent No. 5,527,676, it is stated that tissue samples in which a mutation is to be detected should be enriched for tumor cells in order to detect the loss of heterozygosity in a p53 gene.

Techniques, such as PCR, have been used to detect a loss of heterozygosity resulting from massive deletions characteristic of late-stage adenomas. *See, e.g.*, U.S. Patent No. 5,330,892. Such techniques generally require the use of large numbers of primer pairs, and they will not work at all in a heterogeneous sample. A recent publication reports the use of PCR and ELISA techniques to perform quantitative analysis of mutations in early-stage tumors. U. S. Patent No. 5,512,441. Identification of an abnormal subpopulation of cells in a heterogeneous sample of mostly normal cells and cellular debris, which subpopulation is characterized by loss of heterozygosity or microsatellite instability, is even more difficult because such detection involves the identification of a subpopulation of nucleotide fragments that are difficult to distinguish from the sea of heterogeneous normal cellular material in which they exist. A further problem is that mutation at any locus of a growing number of different oncogenes or tumor suppressor genes can result in cancer, and a screening approach capable of scanning all or even most loci in these genes is not currently available.

Microsatellite instability may also be a marker for cancer. Microsatellites are dispersed throughout the genome at an average frequency of about 1 in 100,000 base pairs. They comprise tandem or trinucleotide repeats that normally are inherited in a stable fashion. *See, e.g.,* Charlesworth, *et al., Nature, 371*: 215-220 (1994). While these sequences perform no known function in the genome, many of them have been mapped and have been used as markers based upon their sequence length polymorphisms. Clonal changes in microsatellite DNA associated with defects in mismatch repair pathways are thought to be suitable markers for hereditary non-polyposis colorectal cancer (HNPCC). In HNPCC tumor samples, for example, Microsatellites were found to have multiple insertions and/or deletions. Microsatellite instability may be an effective marker for failure of mismatch repair in oncogenes or in tumor suppressor genes. While microsatellite instability itself is not indicative of cancer, it is evidence that mutations may occur in regions that are critical for onset of cancer. It follows that detection of instability in Microsatellites indicates that the patient is at risk of developing a clonal subpopulation of cancer cells.

Colorectal cancer is a common cause of death in Western society. Any tumor or precancerous polyp that develops along the length of the colon or the rectum sheds cells or DNA from cells into the lumen of the colon. Shed cells or cellular DNA are usually incorporated on stool as stool passes through the colon. In the early stages of cancer, cancerous or precancerous cells represent a very small fraction of the shed epithelial cells or DNA in stool. Current methods for detection of colorectal cancer do not focus on detecting cancerous or precancerous cells in stool. Rather, such methods typically focus on extracellular indicia of the presence of cancer, such as the presence of fecal occult blood or carcinoembryonic antigen circulating in serum.

It is known, however, that both sporadic and hereditary colorectal cancers result from mutations in oncogenes and tumor suppressor genes. Such mutations appear to occur at a point in the etiology of the disease that is much earlier than the point at which extracellular indicia or clinical signs of cancer are observed. if detected early, colon cancer may be effectively treated by surgical removal of the cancerous tissue. Surgical removal of early-stage colon cancer is usually successful because colon cancer begins in cells of the colonic epithelium and is isolated from the general circulation until the occurrence of invasion through the epithelial lining. Thus, detection of early mutations in colorectal cells would greatly increase survival rate.

Current non-invasive methods for detection of colon cancer involve the detection of fecal occult blood and carcinoembryonic antigen. These screening methods often either fail to detect colorectal cancer or they detect colorectal cancer only after it has progressed to an untreatable stage. Moreover, carcinoembryonic antigen is thought not to be an effective predictor of cancer but merely an indicator of recurrent cancer.

Invasive techniques, such as endoscopy, while effective, are expensive and painful and suffer from low patient compliance. Accordingly, current colon cancer screening methods are not practical for screening large segments of the population. *See, e.g.,* Blum, *Europ. J. Cancer, 31A*: 1369-1372 (1995).

Therefore, there is a need in the art for simple and efficient non-invasive methods for reliable large-scale screening to identify individuals with early stage colon cancer. Such methods are provided herein.

## SUMMARY OF THE INVENTION

The invention relates to a method of detecting the presence of a subpopulation of cells in a biological sample obtained from an organism comprising the steps of:

a) determining from the biological sample a number X of a first wild-type polynucleotide characteristic of a region that is not mutated, in said subpopulation of cells;
b) determining from the biological sample a number Y of a second wild-type polynucleotide suspected of being mutated in said subpopulation of cells; and
c) determining whether a statistically significant difference exists between said number X wherein said number X is indicative of the amount of a reference allele in said sample and said number Y wherein said number Y is indicative of the amount of wild-type target allele in said sample, the presence of a statistically significant difference being indicative of the presence of a subpopulation of cells in said biological sample.

The present invention also provides methods for detecting a subpopulation of genomically transformed cells or cellular debris. Such methods detect the presence in a biological sample of a clonal subpopulation of cells which have a genome different from that of the wild type, and from bacterial, parasitic, or contaminating organisms that may also be present in the sample. Practice of the invention permits, for example, detection of a trace amount of DNA derived from cancer or precancer cells in a biological sample containing a majority of "normal" DNA or whole cells. A preferred use of the methods is to reliably detect in a stool sample voided by a patient the presence of a trace amount of cells and/or cellular debris containing DNA shed into the colon at the site of an asymptomatic precancerous or cancerous lesion. The invention takes advantage of several important insights which permit, for example, reliable detection of a DNA *deletion* at a known genomic site characteristic of a known cancer cell type.

In general, the invention comprises the comparative measurement of two genomic sequences. One genomic sequence is stable through transformation, (i.e., it is identical in both malignant and wild type cells in the sample). A second genomic sequence typically undergoes change during the course of transformation (i.e., it is mutated during the development of malignant precursor cells). Hybridization probes are used to detect the presence of each genomic sequence. If the number of hybridization events involving the two genomic sequences is different, the difference may be due to insignificant background or it may be due to a statistically-significant difference in the quantities of the two genomic sequences in the population from which the sample was drawn. In the latter case, the difference can be correlated, to a degree of defined statistical confidence, with the presence in the sample of a subpopulation of cells having an altered (i.e., non-wild type) genomic sequence.

The invention may be divided into three general embodiments. (1) In a first general embodiment, a quantitative amount (number of copies) of a gene or gene fragment of interest in a sample (i.e. a gene the mutation of which is known or suspected to be associated with cancer) is compared to a quantitative amount of a reference gene or gene fragment in the sample, the reference gene being a gene which is not normally associated with cancer and which normally has a low rate of mutation. A statistically-significant difference between the two quantitative amounts is indicative of genomic instability in a cellular subpopulation in the sample. (2) In a second general embodiment of the invention, a quantitative amount of a region on a maternal allele is compared to a quantitative amount of the corresponding region on a paternal allele. A statistically-significant difference between the two quantitative amounts is indicative of genomic instability. (3) In a third general embodiment, the number of microsatellite repeats at a particular locus are compared between maternal and paternal alleles. A statistically-significant difference in those numbers is indicative of an error in the mismatch repair mechanisms in a subpopulation of cells in the sample or may indicate that allelic loss has taken place. As stated above, errors in mismatch repair may result in mutations in tumor suppressor genes or in oncogenes. Cancer detection in any of the three embodiments of the invention is achieved by measuring the *number* of hybridizations between at least two different nucleotide probes and their respective genomic sequences.

One feature of the invention is that it has now been recognized that materials from cells lining the colon (*e. g.*, a polyp or lesion) are shed onto forming stool only in a region comprising a longitudinal stripe along the length of the stool. Thus, unless the stool sample under investigation is a whole stool or comprises at least a cross-section of a stool, the sample will contain the relevant diagnostic information only by chance. The colon contains numerous bends and folds throughout its length. See, U.S. Patent Application Serial Number, _____ (Atty. Docket No. EXT-002), filed on even date herewith. Epithelial cells lining the colon normally migrate from a basal position in colonic crypts, where stem cells divide by mitosis, to the top of the crypts and are then shed into the lumen. Colonic epithelial cells that line the intestinal lumen typically undergo regeneration every four to five days as a result of the rapid turnover rate through the epithelium. Accordingly, sloughed epithelial cells or their DNA are constantly being deposited in the forming stool as it passes through the lumen. As the stool proceeds toward the rectum and becomes progressively more solid (from an initial liquid state), epithelial cells are only sloughed onto the portion of the stool making contact with the portion of the lumen that formerly contained those cells in its epithelial lining. Epithelial cells of a polyp ( a polyp is a pre-cancerous growth; while not all polyps become cancerous, almost all cancers arise from polyps) undergo the same rapid life cycle and shedding described above for normal colonic epithelial cells. Accordingly, cells shed from polyps are typically only absorbed onto the surface of the forming stool that makes contact with the polyp. However, if the stool is in a liquid state, mixing of shed polyp cells throughout the stool occurs automatically.

Accordingly, the present invention provides methods for detecting genomic changes in a subpopulation of cells in a sample of biological material. Methods of the invention are useful for the detection of changes in the nucleotide sequence of an allele in a small subpopulation of cells present in a large, heterogeneous sample of diagnostically-irrelevant biological material. Methods of the invention are useful for the detection and diagnosis of a genetic abnormality, such as a loss of heterozygosity or, more generally, a mutation, which can be correlated with a disease, such as cancer. For purposes of the present invention, unless the context requires otherwise, a "mutation" includes modifications, rearrangements, deletions, substitutions, and additions in a portion of genomic DNA or its corresponding mRNA.

In a preferred embodiment, the invention provides methods for detecting a clonal subpopulation of transformed cells contained in, or suspected of being contained in, a biological sample obtained from an organism, such as a human. The methods comprise the steps of determining from the biological sample a number X of a first wild-type polynucleotide that is known or suspected not to be mutated in either wild-type cells or in transformed cells. A further step comprises determining from the biological sample a number Y of a second wild-type polynucleotide suspected of being mutated in a subpopulation of cells in the biological sample. Then, one determines whether a statistical-significant difference exists between X and Y. In a normal sample there is no mutation and so there is no statistically-significant difference between the number of each of the somatic genes in a normal cell. As a result, X and Y are not different from each other in a statistically significant sense. In contrast, the presence of a statistically significant difference between X and Y is indicative of the presence of a clonal subpopulation of transformed cells in the biological sample embodying a mutation. Statistical significance may be determined by any method known in the art. However, no formal measurement of statistical significance need be performed in connection with any given assay. Rather, assays are designed to detect a large enough number of binding events such that at least a threshold difference between the numbers is dispositive of the issue of the presence of a mutant subpopulation of cells at any desired level of certainty.

Also, in a preferred embodiment, transformed cells sought to be detected using methods according to the invention are malignant cells. Transformed cells detected according to methods of the invention may be induced transformants, transformed, for example, by a virus, by radiation, or by chemical or other carcinogenic means. Methods of the invention may be performed on any biological sample, including tissue and body fluid samples. Particularly preferred biological samples include pus, sputum, semen, blood, saliva, cerebrospinal fluid, and urine. In an important embodiment of the invention the sample is stool which is analyzed to detect colorectal cancer or precancer. Methods of the invention may be practiced by exposing the biological sample to one or more oligonucleotide probes in order to separately detect the number X of a first polynucleotide and the number Y of a second polynucleotide. Probes for use in the invention are detectably labeled. Preferred labels include fluorescent labels attached, for example, by affinity binding pairs (such as carbohydrate/lectin or avidin/biotin). Highly-preferred labels are microscopic particles which are counted by a detection apparatus, preferably a high-speed electronic apparatus as disclosed herein. The numbers X and Y are preferably proportional and most preferably equal to the number of target polynucleotide detection events occurring in the biological sample.

Methods of the invention are especially useful for the detection of colorectal cancer or precancerous cells in humans. For purposes of the present invention, precancerous cells are cells that have a mutation that is associated with cancer, and which renders such cells susceptible to becoming cancerous. Such methods comprise determining whether cells or nucleotide debris in a stool sample include a deletion of a polynucleotide normally present in a wild-type genome of the human or other mammal. The sample may be exposed to a plurality of first and second oligonucleotide probes under hybridization conditions, thereby to hybridize (i) first probe to copies of a first polynucleotide segment characteristic of a wild-type genomic region known or suspected not to be deleted in cells of the sample and (ii) second probe to copies of a second polynucleotide segment characteristic of the wild-type genomic region suspected of being mutated in the sample. The number of duplexes formed with each of the first and second probes is then detected and counted. The presence of a statistically-significant difference in those two numbers is indicative of the presence in the sample of a mutation that may be characteristic of colorectal cancer. Endoscopy or other visual examination procedures are then indicated.

In a preferred embodiment, probes are labeled with beads or particles. In this embodiment, probes used for detection of genomic polynucleotide segments in a sample are preferably bound to such beads in a ratio of one probe to one bead, and the beads linked to the first and second probes are distinguishable, for example, by size. Use of such hybridization beads or particles facilitates the quantitative detection of genomic polynucleotide segments in the sample using, for example, an impedance counter, such as a "Coulter counter".

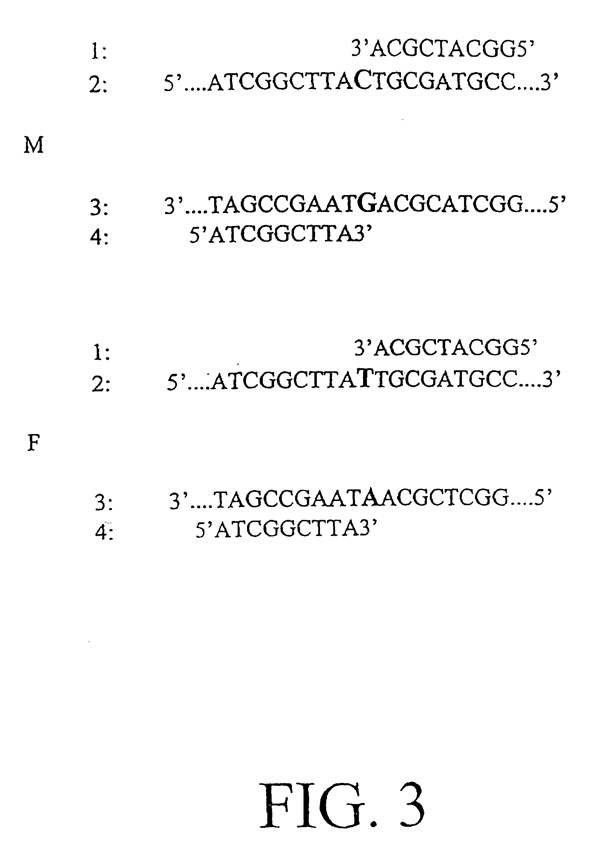

Methods according to the invention also may be used to detect a loss of heterozygosity at an allele by determination of the amounts of maternal and paternal alleles comprising a genetic locus that includes at least one single-base polymorphism. A statistically-significant difference in the amounts of each allele is indicative of a mutation in an allelic region encompassing the single-base polymorphism. In this method, a region of an allele comprising a single-base polymorphism is identified, using, for example, a database, such as GenBank, or by other means known in the art. Probes are designed to hybridize to corresponding regions on both paternal and maternal alleles immediately 3' to the single base polymorphism as shown in Figure 3. After hybridization, a mixture of at least two of the four common dideoxy nucleotides are added to the sample, each labeled with a different detectable label. A DNA polymerase is also added. Using allelic DNA adjacent the polymorphic nucleotide as a template, hybridized probe is extended by the addition of a single dideoxynucleotide that is the binding partner for the polymorphic nucleotide. After washing to remove unincorporated dideoxynucleotides, the dideoxynucleotides which have been incorporated into the probe extension are detected by determining the number of bound extended probes bearing each of the two dideoxy nucleotides in, for example, a flow cytometer or impedance counter. The presence of an almost equal number of two different labels mean that there is normal heterozygosity at the polymorphic nucleotide. The presence of a statistically-significant difference between the detected numbers of the two labels means that a deletion of the region encompassing the polymorphic nucleotide has occurred in one of the alleles.

Methods of the invention may be used to determine whether a patient is a candidate for follow-up invasive diagnostic or other procedures, such as endoscopy. For example, methods of the invention may be used to detect a mutation in a tumor suppressor gene or an oncogene in a subpopulation of cells in a stool sample obtained from a patient. An endoscopy procedure may then be performed on patients diagnosed with a mutation. A positive endoscopy result is then followed by polypectomy, surgery, or other treatment to remove cancerous or precancerous tissue.

Accordingly, it is an object of the invention to provide methods for detecting genomic instability in a subpopulation of cells in a cellular sample. It is a further object of the invention to provide methods for detecting a genomic change in a subpopulation of cells, wherein the genomic change is indicative of cancer. It is another object of the invention to detect a loss of heterozygosity in a genomic region associated with cancer, such as a tumor suppressor region. It is yet another object of the invention to provide methods for detecting heterozygosity and the loss thereof at single-base polymorphic nucleic acids. Finally, it is an object of the invention to provide methods for the detection of cancer, and particularly colorectal cancer by detection of cells or cellular debris indicative of cancer in a heteogeneous sample, such as a stool sample.

Further aspects of the invention will become apparent upon consideration of the following detailed description and of the drawings.

## DESCRIPTION OF THE DRAWINGS

Figure 1 is a flow chart showing sequential steps in methods of the invention.

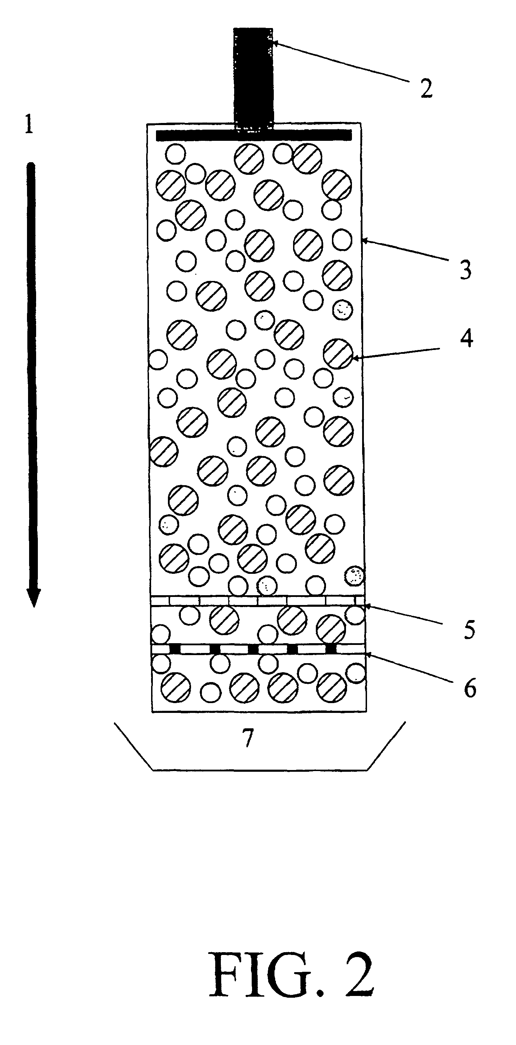

Figure 2 is a schematic diagram of a multi-orifice impedance counter of the type useful in accordance with the invention to count hybridization events; wherein reference numeral 1 indicates the direction of flow through the column; reference numeral 2 indicates a plunger means for forcing material downward in the column; reference numerals 3 and 4 are different-sized hybridization beads; reference numeral 5 is an optional filter for extracting unwanted particles; reference numeral 6 indicates an array of orifices for measuring differential impedance; and reference numeral 7 is a collection chamber.

Figure 3 shows four possible probe attachment sites on allelic regions characterized by having a single base polymorphism. In Figure 3, sequence M1 is SEQ ID NO:1; sequence M2 is SEQ ID NO: 2; sequence M3 is SEQ ID NO:3; sequence M4 is SEQ ID NO: 4; sequence F1 is SEQ ID NO: 5; sequence F2 is SEQ ID NO: 6; sequence F3 is SEQ ID NO: 7; sequence F4 is SEQ ID NO: 8.

Figures 4A and 4B are model Gaussian distributions showing regions of low statistical probability.

Figure 5 is graph showing the probable values of N for a heterogeneous population of cells in which 1% of the cells are mutated.

## DETAILED DESCRIPTION OF THE INVENTION

Methods according to the present invention are useful for the detection of genomic instability in a heterogeneous cellular sample in which the genomic instability occurs in only a small subpopulation of cells in the sample. Using traditional detection methods, such a subpopulation would be difficult, if not impossible, to detect - especially if the mutation that is causative of genomic instability is unknown at the time of detection or a clonally-impure cellular population is used. *See, e.g.,* U.S. Patent No. 5,527,676 (reporting that a clonal population of cells should be used in order to detect a deletion in a p53 gene). Traditional methods for detection of mutations involved in carcinogenesis rely upon the use of a clonally-pure population of cells and such methods are best at detecting mutations that occur at known "hot spots" in oncogenes, such as k-ras. See, Sidransky, supra. Using the PCR-based methods of the art, an extremely large number of primers would have to be designed and sample would have to be tested numerous times to detect genomic instability in a cellular sample that is clonally-impure (*i.e.*, a heterogeneous sample such as stool) and in which the mutation to be detected is unknown and exists in a very small number of cells. Moreover, PCR is not useful for detection of the absence of a genetic sequence, as in the case of the present methods for detecting loss of heterozygosity. Even after such repeated testing, a PCR-based method may not detect a mutation in a small number of cells in a clonally-impure population if, for example, primers bordering the site of the mutation are not used. Thus, in early-stage adenomas (when the population of mutated cells is very small), methods of the art are, at best, impractical and may not work at all.

In contrast, methods of the present invention are capable of detecting genomic instability in a small number of cells in an impure cellular population because such methods do not rely upon knowing which mutation exists and such methods are not affected by the presence in the sample of heterogeneous DNA. For example, in loss of heterozygosity, deletions occur over large portions of the genomes and entire alleles may be missing (or at least enough of an allele may be missing in order to render the allele non-functional). Methods of the invention comprise counting a number of a gene suspected of being mutated and comparing that number with the number of a gene known not to be mutated in the same sample. All that one needs to know is at least a portion of the sequence of a wild-type gene in which the mutation is suspected to occur and at least a portion of the wild-type sequence of a reference gene in which mutation is not suspected to have occurred.

Accordingly, methods of the present invention are useful for the detection of changes in a genomic nucleotide sequence present in a subpopulation of cells or debris therefrom in a sample. Such changes generally occur as a mutation (i.e., a substitution, modification, deletion, addition, or rearrangement) in a wild-type allelic sequence in a subpopulation of cells. In the case of a tumor suppressor gene, the mutation typically takes the form of a massive deletion characteristic of loss of heterozygosity. Often, as in the case of certain forms of cancer, disease-causing mutations initially occur in a single cell which then produces a small subpopulation of mutant cells. By the time clinical manifestations of the mutation are detected, the disease may have progressed to an incurable stage. Methods of the invention allow detection of a mutation when the mutation exists as only a small percentage of the total cells or cellular debris in a sample.

Methods of the invention comprise a comparison of two wild-type sequences that are expected to be present in the sample in equal numbers in normal (non-mutated) cells. In a preferred embodiment, the comparison is between (1) an amount of a genomic polynucleotide segment that is known or suspected not to be mutated in cells of the sample (the "reference") and (2) an amount of a wild-type (non-mutated) genomic polynucleotide segment suspected of being mutated in a subpopulation of cells in the sample (the "target"). A statistical-significant difference between the amounts of the two genomic polynucleotide segments indicates that a mutation has occurred. Specifically, in the case of a deletion in a tumor suppressor gene, the detected amount of the reference gene is significantly greater than the detected amount of the target gene. If a target sequence is amplified, as in the case of certain oncogene mutations, the detected amount of target is greater than the detected amount of the reference gene by a statistically-significant margin.

Methods according to the art generally require the use of numerous probes, usually in the form of PCR primers and/or hybridization probes, in order to detect a deletion or a point mutation. However, because methods of the present invention involve quantitative detection of nucleotide sequences and quantitative comparisons between sequences that are known to be stable and those that are suspected of being unstable, only a few probes must be used in order to accurately assess cancer risk. In fact, a single set (pair) of probes is all that is necessary. The risk of cancer is indicated by the presence of a mutation in a genetic region known or suspected to be involved in oncogenesis. Patients who are identified as being at risk based upon tests conducted according to methods of the invention are then directed to other, typically invasive, procedures for confirmation and/or treatment of the disease.

Quantitative sampling of a nucleotide sequence that is uniformly distributed in a biological sample typically follows a Poisson distribution. For large populations, such as the typical number of genomic polynucleotide segments in a biological sample, the Poisson distribution is similar to a normal (Gaussian) curve with a mean, N, and a standard deviation that may be approximated as the square root of N.

Statistically-significance between numbers of target and reference genes obtained from a biological sample may be determined by any appropriate method. *See, e.g.*, Steel, *et al.*, Principles and Procedures of Statistics, A Biometrical Approach (McGraw-Hill, 1980). An exemplary method is to determine, based upon a desired level of specificity (tolerance of false positives) and sensitivity (tolerance of false negatives) and within a selected level of confidence, the difference between numbers of target and reference genes that must be obtained in order to reach a chosen level of statistical significance. A threshold issue in such a determination is the minimum number, N, of genes (for each of target and reference) that must be available in a population in order to allow a determination of statistical significance. The number N will depend upon the assumption of a minimum number of mutant alleles in a sample containing mutant alleles (assumed herein to be at least 1%) and the further assumption that normal samples contain no mutant alleles. It is also assumed that a threshold differences between the numbers of reference and target genes must be at least 0.5% for a diagnosis that there is a mutation present in a subpopulation of cells in the sample. Based upon the foregoing assumptions, it is possible to determine how large N must be so that a detected difference between numbers of mutant and reference alleles of less than 0.5% is truly a negative (i.e. no mutant subpopulation in the sample) result 99.9% of the time.

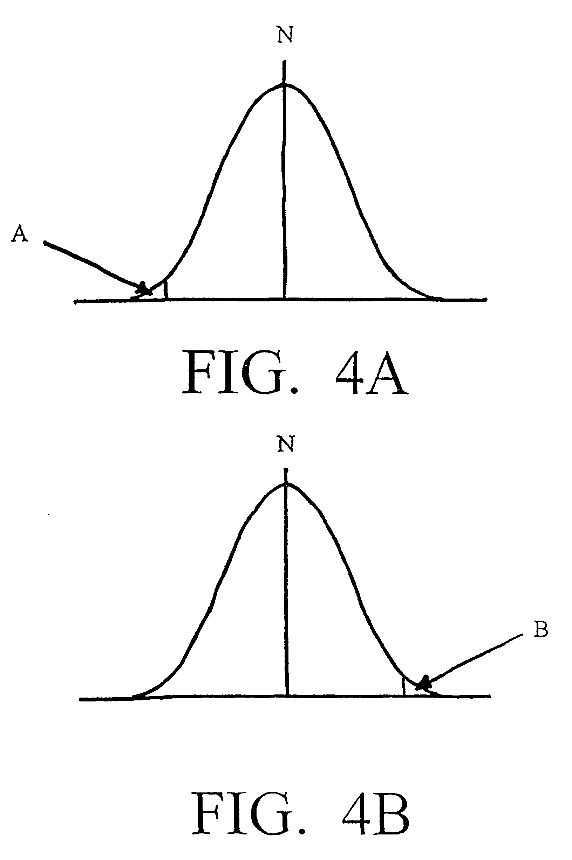

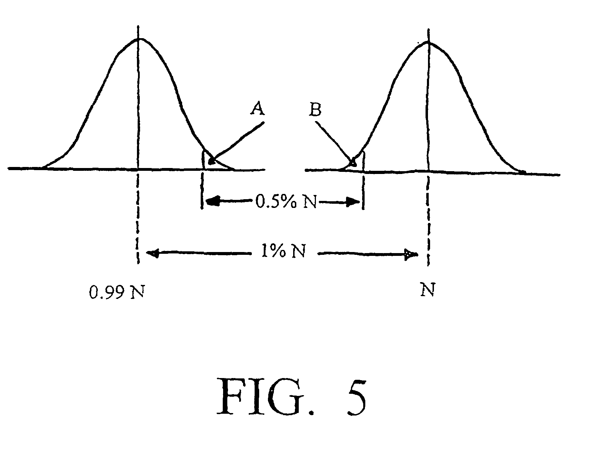

The calculation of N for specificity, then, is based upon the probability of one sample measurement being in the portion of the Gaussian distribution covering the lowest 3.16% of the population (the area marked "A" in figure 4A) and the probability that the other sample measurement is in the portion of the Gaussian distribution covering the highest 3.16% of the population (the area marked "B" in figure 4B). Since the two sample measurements are independent events, the probability of both events occurring simultaneously is approximately 0.001 or 0.1%. Thus, 93.68% of the Gaussian distribution (100% - 2x3.16%) lies between the areas marked A and B in figure 5A. Statistical tables indicate that such area is equivalent to 3.72 standard deviations. Accordingly, 0.5%N equals 3.72 sigma. Since sigma (the standard deviation) is equal to $\sqrt{N}$, the equation may be solved for N as 553,536. This means that if the lower of the two numbers representing reference and target is at least 553,536 and if the patient is normal, the difference between the numbers will be less than 0.5% about 99.9% of the time.

To determine the minimum N required for 99% sensitivity a similar analysis is performed. This time, one-tailed Gaussian distribution tables show that 1.28 standard deviations (sigma) from the mean cover 90% of the Gaussian distribution. Moreover, there is a 10% (the square root of 1%) probability of one of the numbers (reference or target) being in either the area marked "A" in figure 5 or in the area marked "B" in figure 5. If the two population means are a total of 1% different and if there must be a 0.5% difference between the number of target and reference genes, then the distance from either mean to the threshold for statistical significance is equivalent to 0.25%N (See Figure 5) for 99% sensitivity. As shown in Figure 5, 0.25%N corresponds to about 40% of one side of the Gaussian distribution. One-tailed statistical tables reveal that 40% of the Gaussian distribution corresponds to 1.28 standard deviations. Therefore, 1.28 sigma is equal to 0.0025N, and N equals 262,144. Thus, for abnormal samples, the difference will exceed 0.5% at least 99% of the time if the lower of the two numbers is at least 262,144. Conversely, an erroneous negative diagnosis will be made only 1% of the time under these conditions.

In order to have both 99.9% specificity (avoidance of false positives) and 99% sensitivity (avoidance of false negatives), a sample with at least 553,536 (or roughly greater than 550,000) of both target and reference alleles should be used. A difference of at least 0.5% between the numbers obtained is significant at a confidence level of 99.0% for sensitivity and a difference of less than 0.5% between the numbers is significant at a confidence level of 99.9% for specificity. As noted above, other standard statistical tests may be used in order to determine statistical significance and the foregoing represents one such test.

Based upon the foregoing explanation, the skilled artisan appreciates that methods of the invention are useful to detect mutations in a subpopulation of a polynucleotides in any biological sample. For example, methods disclosed herein may be used to detect allelic loss (the loss of heterozygosity) associated with diseases such as cancer. Additionally, methods of the invention may be used to detect a deletion or a base substitution mutation causative of a metabolic error, such as complete or partial loss of enzyme activity. For purposes of exemplification, the following provides details of the use of methods according to the present invention in colon cancer detection. Inventive methods are especially useful in the early detection of a mutation (and especially a large deletion typical of loss of heterozygosity) in a tumor suppressor gene.

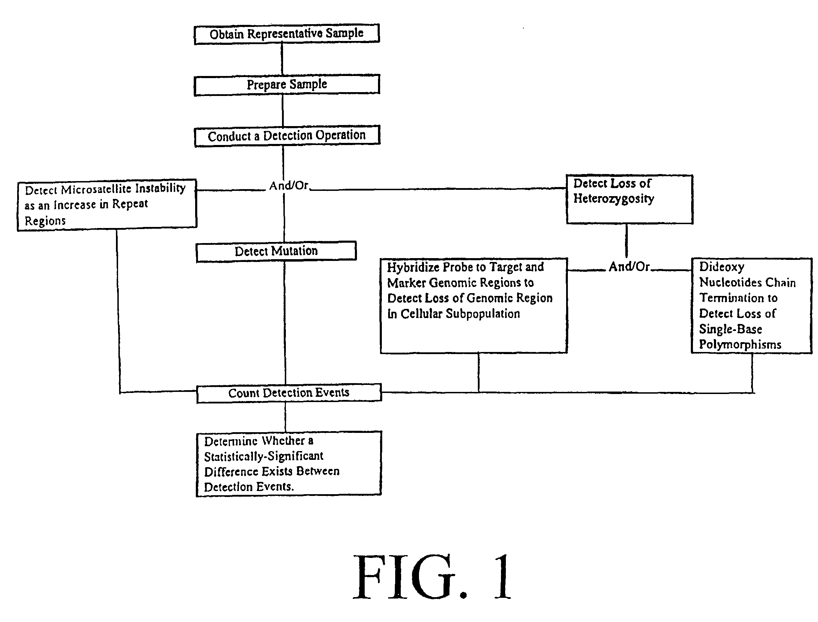

Methods according to the invention preferably comprise one of three types of detection regimens. In a first preferred detection regimen, an amount of a polynucleotide known or suspected to be mutated is compared to an amount of a reference polynucleotide known or suspected not to be mutated. In a second preferred detection regimen, an amount of a polymorphic nucleotide on a maternal allele is compared to an amount of the corresponding polymorphic nucleotide on the corresponding paternal allele. Finally, a third detection regimen comprises a comparison of a microsatellite repeat region in a normal allele with the corresponding microsatellite region in an allele known or suspected to be mutated. All three exemplary detection means comprise determining whether a difference exists between the amounts of each nucleic acid being measured. The presence of a statistically-significant difference is indicative that a mutation has occurred in one of the nucleic acids being measured. Thus, methods described below are generally applicable to all forms of the invention, the variations of which are shown in figure 1.

**I. Preparation of a Stool Sample**

A sample prepared from stool voided by a patient should comprise at least a cross-section of the voided stool. As noted above, stool is not homogenous with respect to sloughed cells. As stool passes through the colon, it absorbs sloughed cells from regions of the colonic epithelium with which it makes contacts. Thus, sloughed cells from a polyp are absorbed on only one surface of the forming stool (except near the cecum where stool is still liquid and is homogenized by Intestinal Peristalsis). Taking a representative sample of stool (i.e., at least a cross-section) and homogenizing it ensures that sloughed cells from all epithelial surfaces of the colon will be present for analysis in the processed stool sample. Stool is voided into a receptacle that is preferably small enough to be transported to a testing facility. The receptacle may be fitted to a conventional toilet such that the receptacle accepts stool voided in a conventional manner. The receptacle may comprise a mesh or a screen of sufficient size and placement such that stool is retained while urine is allowed to pass through the mesh or screen and into the toilet. The receptacle may additionally comprise means for homogenizing voided stool. Moreover, the receptacle may comprise means for introducing homogenization buffer or one or more preservatives, such as alcohol or a high salt concentration solution, in order to neutralize bacteria present in the stool sample and to inhibit degradation of DNA.

The receptacle, whether adapted to fit a toilet or simply adapted for receiving the voided stool sample, preferably has sealing means sufficient to contain the voided stool sample and any solution added thereto and to prevent the emanation of odors. The receptacle may have a support frame which is placed directly over a toilet bowl. The support frame has attached thereto an articulating cover which may be placed in a raised position, for depositing of sample or a closed position (not shown) for sealing voided stool within the receptacle. The support frame additionally has a central opening traversing from a top surface through to a bottom surface of the support frame. The bottom surface directly communicates with a top surface of the toilet. Extending from the bottom surface of the support frame and encompassing the entire circumference of the central opening is a means for capturing voided stool. The means for capturing voided stool may be fixedly attached to the support frame or may be removably attached for removal subsequent to deposition of stool.

Once obtained, the stool sample is homogenized in an appropriate buffer, such as phosphate buffered saline or a chaotropic salt solution. Homogenization means and materials for homogenization are generally known in the art. See, e.g., U.S. Patent No. 4,101,279. Thus, particular homogenization methods may be selected by the skilled artisan. Methods for further processing and analysis of a biological sample, such as a stool sample are presented below.

**II. Methods for Detection of Colon Cancer or Precancer**

A. Reference-Target

For exemplification, methods of the invention are used to detect a deletion or other mutation in the p53 tumor suppressor gene in cells obtained from a representative stool sample. The p53 gene is a good choice because the loss of heterozygosity in p53 is often associated with colorectal cancer. An mRNA sequence corresponding to the DNA coding region for p53 is reported as GenBank Accession No. M92424. The skilled artisan understands that methods described herein may be used to detect mutations in any gene and that detection of a p53 deletion is exemplary of such methods. At least a cross-section of a voided stool sample is obtained and prepared as described immediately above. DNA or RNA may optionally be isolated from the sample according to methods known in the art. See, Smith-Ravin, *et al.*, *Gut*, *36*: 81-86 (1995), However, methods of the invention may be performed on unprocessed stool.

Nucleic acids may be sheared or cut into small fragments by, for example, restriction digestion. The size of nucleic acid fragments produced is not critical, subject to the limitations described below. A target allele that is suspected of being mutated (p53 in this example) and a reference allele are chosen. A reference allele may be any allele known or suspected not to be mutated in colon cancer cells. Single-stranded nucleic acid fragments may be prepared using well-known methods. See, e.g., Sambrook, *et al., Molecular Cloning, A Laboratory Manual* (1989).

Either portions of a coding strand or its complement may be detected in methods according to the invention. For exemplification, detection of the coding strand of p53 and reference allele are described. Complement to both p53 and reference allele are removed by hybridization to anti-complement oligonucleotide probes (isolation probes) and subsequent removal of duplex formed thereby. Methods for removal of complement strands from a mixture, of single-stranded oligonucleotides are known in the art and include techniques such as affinity chromatography. Upon converting double-stranded DNA to single-stranded DNA, sample is passed through an affinity column comprising bound isolation probe that is complementary to the sequence to be isolated away from the sample. Conventional column chromatography is appropriate for isolation of complement. An affinity column packed with sepharose or any other appropriate materials with attached complementary nucleotides may be used to isolate complement DNA in the column, while allowing DNA to be analyzed to pass through the column. *See* Sambrook, *Supra.* As an alternative, isolation beads may be used to exclude complement as discussed in detail below.

After removal of complement strands, first oligonucleotide probes which hybridize to at least a portion of the p53 allele and second oligonucleotide probes that hybridize to at least a portion of the reference allele are obtained. The probes are laceled with a detectable label, such as fluorescein or detectable particles. Distinct labels for the probes are preferred.

Labeled probes are then exposed to sample under hybridization conditions. Such conditions are well-known in the art. *See, e.g.*, Wallace, et al., *Nucleic Acids Res., 6*:3543-3557 (1979), First and Second oligonucleotide probes that are distinctly labeled (i.e. with different radioactive isotopes, fluorescent means, or with beads of different size, See *infra*) are applied to a single aliquot of sample. After exposure of the probes to sample under hybridization conditions, sample is washed to remove any unhybridized probe. Thereafter, hybridized probes are detected separately for p53 hybrids and reference allele hybrids. Standards may be used to establish background and to equilibrate results. Also, if differential fluorescent labels are used, the number of probes may be determined by counting differential fluorescent events in a sample that has been diluted sufficiently to enable detection of single fluorescent events in the sample. Duplicate samples may be analyzed in order to confirm the accuracy of results obtained.

If there is a statistically-significant difference between the amount of p53 detected and the amount of the reference allele detected, it may be assumed that a mutation has occurred in p53 and the patient is at risk for developing or has developed colon cancer. Statistical significance may be determined by any known method. A preferred method is outlined above.

The determination of a p53 mutation allows a clinician to recommend further treatment, such as endoscopy procedures, in order to further diagnose and, if necessary, treat the patient's condition. The following examples illustrate methods of the invention that allow direct quantification of hybridization events.

*1. Method for Increased Quantitation of Target and Reference Polynucleotides*

Enhanced quantification of binding events between hybridization probes and target or reference is accomplished by coupling hybridization probes to particles, such as beads (hybridization beads).

In order to obtain a precise quantitative measure of the amount of a polynucleotide in a sample, hybridization beads are constructed prior to conducting hybridizations, such that each bead has attached thereto a single oligonucleotide probe.

*a. Method for Preparation of Probe-Bead Combinations*

A single probe is attached to a bead by incubating a large excess of hybridization beads with oligonucleotide probes of a given type (*i.e.*, either first or second oligonucleotide probes). Coupling of probe to bead is accomplished using an affinity-binding pair. For example, beads may be coated with avidin or streptavidin and probes may be labeled with biotin to effect attachment of the probe to the bead. The mixture of beads and probes is agitated such that virtually 100% of the probes are bound to beads. The mixture is then exposed to a matrix, such as an affinity column or a membrane coated with oligonucleotides that are complementary to the probe. Only beads that have an attached probe will adhere to the matrix, the rest being washed away. Beads with coupled probe are then released from the matrix by melting hybridizations between probe and complement. Multiple exposures to the matrix and pre-washing of the column reduces non-specific binding. Moreover, naked beads (*i.e.*, without attached probe) may be exposed to the matrix to determine a background number of beads that can be expected to attach to the matrix in the absence of probe.

By using a vast excess of beads relative to probe as described above, it is expected that the vast majority of recovered beads will have only one attached probe. For example, if a mixture has a ratio of 1 probe to 1000 beads, it is expected that only about 1 bead in a million will have two attached probes and even less than one bead in a million will have more than two attached probes. Accordingly, hybridization beads are provided in an effective 1:1 ratio with probe which allows for precise quantification of target and reference polynucleotide as described below.

For each assay described below, two distinct hybridization beads are used. A first hybridization bead has attached thereto a single first oligonucleotide probe that is complementary to at least a portion of a target polynucleotide (*e.g.*, a p53 allele). A second hybridization bead, of a size distinct from the first hybridization bead, has attached thereto a single second oligonucleotide probe that is complementary to at least a portion of a reference polynucleotide (*i.e.*,

one that is known or suspected not to be mutated in the sample).

*b. Use of Beads to Quantify Target and Reference Polynucleotides*

DNA is melted (denatured to form single-stranded DNA) by well-known methods *See, e.g.,* Gyllensten, *et al.*, *in Recombinant DNA Methodology II*, 565-578 (Wu, ed., 1995). According to methods of the invention, one may detect either a coding strand or its complement in order to quantify target and/or reference polynucleotide. For purposes of illustration, the present example assumes detection of the coding strand.

*2. Removal of Complement*

Single-stranded complement of the target polynucleotide (e.g., p53) and reference polynucleotide are removed from the sample by binding to oligonucleotide probes that are complementary to target or reference complement. Such probes, referred to herein as isolation probes, are attached to isolation beads prior to their introduction into the sample. The beads may be magnetized. Thus, when magnetized isolation beads [with attached isolation probe(s)] are introduced into the sample, the attached isolation probes hybridize to complement of target or reference (or vice versa). Isolation beads are preferably introduced in vast excess in order to saturate complement binding. Once hybridization is complete, a magnetic field is applied to the sampte, thus drawing the magnetized isolation beads (both with and without hybridized complement) out of the sample. Assuming that a sufficient quantity of isolation beads are introduced into the sample, removal of the isolation beads effectively removes all target and reference complement from the sample. In an alternative embodiment for complement removal, an excess of oligonucleotide probe with attached biotin is exposed to the dehybridized sample under hybridization conditions. Once hybridization is complete the sample is exposed to a column lined with avidin. The biotin-bound probe, whether free or hybridized to complement, is bound by avidin on the column. The remainder of the DNA, including target and reference coding strands to be detected, is passed through the column. In contrast to the description of hybridization beads above, beads for removal of complement may be constructed so numerous oligonucleotides are bound to a single bead.

*3. Detection and Quantitation of Target and Reference*

Two sets of hybridization beads are prepared as described above. Each member of a first set of hybridization beads (all of which are identical to each other) has attached thereto a single oligonucleotide probe that is complementary to at least a portion of the target polynucleotide. Each member of a second set of identical hybridization beads (all of which are identical to each other but not to the first set) has attached thereto a single oligonucleotide probe that is complementary to at least a portion of the reference polynucleotide. Members of the second set of hybridization beads are of a size or color distinct from that of members of the first set of hybridization beads. First and second hybridization beads may also be distinguished on the basis of other characteristics. For example, beads may have attached fluorescent markers that are distinguished by their fluorescence at different wavelengths. Beads with distinct electrochemical charges may also be used. The precise modality used for distinguishing beads is not essential to the invention as long as it is possible to distinguish between first and second probe on the basis of distinctions between attached first and second beads.

Both sets of hybridization beads with attached probes are exposed to the sample under hybridization conditions, thereby allowing attached probe to hybridize to reference or target. Once hybridization is complete, the sample is washed to remove unhybridized bead/probe combinations. Unhybridized bead/probe combinations are removed by, for example, passing the sample through a column comprising DNA complementary to the probe sequence. Thus, any unhybridized bead/probe combinations are retained on the column while duplex is passed through the column. Subsequently, the sample is exposed to means for differentially counting hybridization beads in order to quantify first and second hybridization probes which have formed duplexes. The numbers obtained provide a precise estimate of the number of copies of the reference and target polynucleotide in the population because differential counting means count individual beads. One bead is equal to one probe which, in turn, signifies one copy of the nucleic acid being measured.

An example of a differential counting means is an impedance measuring device, such as a Coulter counter (Coulter Electronics, Inc., Miami, Florida). Sample is passed through the device which differentially detects the two types of hybridization beads by measuring their differential impedance of an electric current. Alternatively, the device may measure fluorescent, color, or other changes. In order to increase the speed of the assay, a multi-orifice device may be used. A multi-orifice impedance counter is shown schematically in figure 2. A multi-orifice array is placed at one end of a column filled with an electrically-conductive fluid, such as saline. Hybridization beads with either hybridized target or reference segments are inserted at an opposite end of the column. Each orifice is large enough to accommodate only one hybridization bead at a time and sufficiently wide to allow reliable impedance measurements. An electric voltage is passed across each orifice. Each hybridization bead (which is nonconducting), as it passes through one of the orifices, displaces a volume of saline, thus creating a brief impedance change that is proportional to the size of the bead. This, in turn, creates a measurable decrease in current that is directly correlated with the size of the bead. By compiling the number of each of the two distinct impedance events, a precise estimate of the number of hybridization beads and, therefore, the number of probes of each type in the population is obtained.

Upon quantitative measurement of first and second hybridization beads, data are analyzed as discussed above to determine whether any statistically-significant difference exists between the amounts of first and second hybridization beads (with hybridized probe attached). A statisticatty-significant reduction in the amount of target is indicative of a mutation in the target allele. Where the p53 gene is the target allele, such a mutation is indicative of a cancerous or precancerous condition. A clinician may use such results as a basis for prescribing additional treatment, such as endoscopy and polypectomy procedures.

B. Detection of Mutations in Single-base Polymorphisms

The basic method described above may also be applied to detect a loss of heterozygosity or other mutation at a single base polymorphic site between maternal and paternal alleles. Such detection is typically an indication of a larger deletion or other mutation. However, a mutation at a single polymorphic nucleotide may be all that is necessary to inhibit gene function in one of the two alleles. A deletion associated with loss of heterozygosity may be difficult to detect due to a recently-discovered phenomenon called complementary reduplication. In complementary reduplication, the loss of one of two alleles at a particular locus results in "reduplication" of the surviving allele. Reduplication usually takes place on the chromosome containing the surviving allele and involves the production of one or more copies of the surviving allele in close proximity on the chromosome to the position of the surviving allele. In the case of a locus that displays one or more single-base allelic polymorphisms (*i.e.*, heterozygosity at the locus is determined by virtue of one or more single-base differences in one or more regions of the locus), complementary reduplication results in the insertion on the chromosome containing the surviving allele of a duplicate of the sequence corresponding to that which was deleted. Even under the most stringent hybridization conditions, some of a probe directed against the deleted sequence will bind to the reduplicated sequence at a locus of a single-base polymorphism. Accordingly, in such circumstances, the deletion may not be detected because any true difference in the number of probes binding to the polymorphic site (*i.e.*, the allelic region encompassing the single-base polymorphism) may be obscured by an increase resulting from the other allele's reduplicated region.

The problems associated with complementary reduplication, and with non-specific probe binding generally, are solved by methods of the invention. Such methods allow detection of a deletion in one of two alleles present at a specific locus in a subpopulation of cells contained in a biologicai sample. Numerous alleles, including tumor suppressor alleles, contain single polymorphic nucleotides in the context of a constant nucleic acid region. Individuals normally may be either homozygous or heterozygous for a particular polymorphic nucleotide. Since numerous single-base polymorphic nucleotide sites exist in most alleles, the probability that a given individual is heterozygous at least one of the single-base polymorphism sites is high. A statistically-significant reduction in one of the two nucleotides at a single-base polymorphic site (at which the individual is heterozygous) is used as a marker for a deletion in the allele encompassing that site.

Genomic regions containing known single-base polymorphisms are identified by reference to a nucleotide database, such as GenBank, EMBL, or any other appropriate database. For purposes of the invention, a single-base polymorphism is intended to be a single polymorphic nucleotide adjacent to a non-polymorphic region of the allele regardless of whether the single polymorphic nucleotide forms part of a larger polymorphic site (i.e. the single-base polymorphism may be the terminal nucleotide of a larger, polynucleotide polymorphism). For cancer detection, the regions considered are regions in which loss of heterozygosity is prevalent, such as tumor suppressor genes. A given individual may be homozygous or heterozygous for the polymorphic nucleotide in any identified single-base polymorphic region. Accordingly, if a number of single-base polymorphic regions are identified, the probability increases that at least one heterozygous single-base polymorphic region is found in a sample.

Once single-base polymorphic sites are identified, a sample is obtained from a patient in order to determine which of those sites is heterozygous in normal (*i.e.*, non-cancerous or pre-cancerous) cells. Then, sample is prepared as described above. Double-stranded DNA in the sample is converted to single-stranded DNA. Then, either the coding strand or the anti-coding strand for both alleles is isolated from the sample. As will be evident from the following discussion, methods disclosed herein are indifferent as to whether coding strand or anti-coding strand is retained in the sample.

An oligonucleotide probe is constructed that is complementary to a portion of the region of single-base polymorphism, said portion ending at the nucleotide that is immediately 3' to the polymorphic nucleotide, regardless of whether the 5'-3' (coding) strand or the 3'-5' (anticoding) strand is used as a template. Figure 3 shows four possible probes that are immediately 3' to the polymorphic nucleotide for each of four possible template strands as described above (the Sequences in Figure 3 are hypothetical and are not intended to represent any actual sequence). While either strand may be used as a template for probe binding to determine heterozygosity and/or the loss thereof, the sequence of the probe that is hybridized to the template will be different depending upon the strand used. Probes may be of any length that allows efficient and specific hybridization of probe to target. Figure 3 illustrates the four probes that are useful for hybridization to the hypothetical sequence shown. The length of probe sequences may be determined as appropriate for each genomic region that is analyzed. A preferable length is between about 10 and about 100 nucleotides. The size of the probe will also depend upon the size of the region surrounding the single-base polymorphism (*i.e.*, the region 5' or 3' to the next adjacent polymorphism, if any). Details concerning the construction and hybridization of oligonucleotide probes are known in the art.

Once constructed, unique probes for each polymorphic region will hybridize to regions of both maternal and paternal alleles up to, but not including, the polymorphic nucleotide, which, in a heterozygote, will be different in the maternal and paternal alleles. Figure 3 illustrates the foregoing hybridization procedure. Figure 3 shows only a small portion of the region surrounding the polymorphic nucleotide. The alleles shown in Figure 3 are heterozygous at the polymorphic site (shown in bold type in Figure 3).

Probe is hybridized to its specific template DNA (see above) by standard methods in the art. The sample may optionally be washed to remove unhybridized probe. To determine whether each single-base polymorphic region to which probe has bound is heterozygous or homozygous at the polymorphic nucleotide, a modification of the dideoxy chain termination method as reported in Sanger, *Proc. Nat'l-Acad. Sci. (USA), 74:* 5463-5467 (1977), is used. The method involves using at least two of the four common 2', 3'-dideoxy nucleoside triphosphates (ddATP, ddCTP, ddGTP, and ddTTP). A different detectable label is attached to each dideoxy nucleoside triphosphate (ddNTP) according to methods known in the art. Differentially-labeled ddNTPs are available from Perkin Elmer Corporation (Cat. No. 401456). At least two labeled ddNTPs then are exposed to each sample having probe hybridized to maternal and paternal alleles as described above. The choice of which two ddNTPs are used will depend upon the nucleotides at the heterozygous polymorphic site. A DNA polymerase, such as Sequenase™ (Perkin-Elmer), is also added to the sample mixture. Using the allelic strands as primer, the polymerase will add one ddNTP to the 3' end of the probe, the incorporated ddNTP being complementary to the nucleotide that exists at the single-base polymorphic site. Because the ddNTPs have no 3' hydroxyl, further elongation of the hybridized probe will not occur. After completion, the sample is washed to remove excess ddNTPs. Label is then counted in each sample. The presence of two differentially-labeled ddNTPs in a sample is indicative of heterozygosity at the polymorphic site. Any 3' modified nucleoside triphosphate may be used in the above-described methods as long as the 3' modification prevents binding of an additional 3' nucleotide (i.e. probe extension) and does not inhibit binding of the modified nucleotide to the 3' end of the probe.

It is not necessary to determine the amount of each label present in the sample in order to establish heterozygosity or homozygosity. For Example, differentially-labeled deoxynucleoside triphosphates may be used for a determination of heterozygosity or homogzygosity. The mere fact that two different labeled dideoxy nucleotides are incorporated into the probe means that the single-base polymorphic site being analyzed is heterozygous. However, determination of sites at which a patient is polymorphic is useful in order to establish a baseline of polymorphisms which may be used in future tests to detect changes in polymorphic sites which may be indicative of cancer. The existence of polymorphisms may be determined by methods taught herein, gel electrophoresis or by other standard methods.

In the case in which heterozygosity exists at the polymorphic site, counting the amount of each of the two differentially-labeled ddNTPs allows a determination of whether there is a loss of heterozygosity (*i.e.*, a deletion) in a subpopulation of cells in the sample. In a normal (*i.e.*, non-cancerous) sample containing cells that are heterozygous at the single-base polymorphic site, it is expected that the detected amount of each of the two ddNTPs added to the probe will be identical (within chosen limits of statistical significance). However, if a deletion has occurred in one of the two alleles in a subpopulation of cells in the sample, there will be a statistically-significant difference between the amounts of each of the two alleles detected via the incorporated (labeled) ddNTPs. The detection of such a difference is indicative of genomic instability within the sample. Such genomic instability indicates the possibility of cancerous or pre-cancerous cells in the sample.

In order to improve the ability to accurately count alleles to which ddNTPs have attached, ddNTPs are labeled with hybridization-type beads of different sizes as described above. Alleles with bound probe comprising a labeled ddNTP are counted as described above using a counting device, such as a Coulter counter. Also as described above, differential fluorescent labels or other counting means may be used to separately detect incorporated ddNTPs.

The detection of heterozygosity at single-base polymorphic sites and the detection of the loss of heterozygosity may be determined in separate steps. For example, probes may be hybridized immediately adjacent to but not including the nucleotide determined to be polymorphic as described above. The four ddNTPs may then be added to the sample, washed, and the presence or absence of each label may be detected. Detection of only one label indicates that the individual from whom the sample was obtained is homozygous at the site of the polymorphic nucleotide. Detection of two labels means that the individual is heterozygous. The heterozygous loci are noted. As noted above, baseline determinations of heterozygosity may be done using standard deoxynucleotides. Once a baseline is established, future tests on that individual are performed on the heterozygous loci in order to detect a loss of heterozygosity as described immediately above. For the detection of cancer, the heterozygous loci are typically tumor suppressor genes, including p53, dcc, apc, and others. Using methods of the invention, a "fingerprint" of heterozygous tumor suppressor loci may be constructed. Future deviation from the fingerprint (*i.e.*, deletions) provides valuable information as to the development of cancer.

A preferred use of the foregoing methods is in the detection of colon cancer. A representative stool sample is prepared as described above. At least a cross-section of the stool sample is placed in buffer and homogenized. Double-stranded DNA is converted to single-stranded DNA and complement of the strand to be detected is removed from the sample by any of the methods described above. The remaining single-stranded DNA is exposed to multiple copies of a probe designed on the basis of known single-base polymorphisms in a cancer-associated allele, such as a tumor suppressor allele, such that the probe hybridizes with a desired number of nucleotides immediately adjacent to the polymorphic nucleotide as described above. After hybridization is complete, the sample is washed and exposed to differentially-labeled ddNTPs and a DNA polymerase. The sample is then washed to remove unincorporated ddNTPs. The presence of any labeled ddNTPs is determined. If two labels are detected, the individual from whom the sample is obtained is heterozygous at the polymorphic nucleotide. The heterozygosity of the allele and the probe sequence matching the site immediately adjacent to the polymorphic allele are noted for reference in future testing for the loss of heterozygosity. Alternatively, once the patient is determined to be heterozygous at a locus, an assay may be performed immediately in the manner described above in order to determine a present loss of heterozygosity in a subpopulation of cells in the sample.

C. Analysis of Microsatellite Instability

Microsatellites are di- or trinucleotide repeats found throughout the genome. A particular array of microsatellite repeats is often associated with a particular genomic sequence and is stably inherited under normal conditions. Expansions of microsatellite copy number typically are associated with defects in mismatch repair. Accordingly, changes in a microsatellite regions indicate that the patient is at risk for a mutation in other genomic regions, which may lead to cancer.

In order to detect microsatellite instability as an indicator of a mutation in a cancer-associated gene, one must first identify a microsatellite region associated with the gene of interest. Such regions are typically identified on a database, such as GenBank, EMBL, and others. Once a wild-type microsatellite region associated with, for example, the p53 tumor suppressor gene is identified, an oligonucleotide probe is constructed that spans the microsatellite region and the regions immediately 5' and immediately 3' to the microsatellite region. The precise length of probes may be determined by the experimenter. Probes are constructed that hybridize to the microsatellite region, including 5' and 3' extensions, on both the maternal and paternal alleles in which the microsatellite is associated with, for example, p53.

An appropriate sample of body tissue or fluid is obtained and processed as described herein. Double stranded DNA is denatured and an excess of maternal and paternal probes, as described above, are introduced into the sample under hybridization conditions. The probes are detectably labeled as described above. Complement of the strands to be detected may optionally be removed by methods described above. The sample is then washed to remove unhybridized probe and the amount of hybridized probe in quantitatively detected.

Quantitative detection may be accomplished by any means described herein. For example, probes may be attached to hybridization beads such that probes that bind to maternal allele are attached to beads of one size and probes that bind to paternal allele are attached to beads of a second size that is distinguishable from beads of the first size. Beads with attached probe may be counted as described above.

The detection of a statistically-significant difference between the amount of probe binding to the maternal allele and the amount of probe binding to the paternal allele is indicative of microsatellite instability. As previously mentioned, microsatellite instability is indicative of a mutation at the locus in which the microsatellite resides. If the microsatellite region is associated with a tumor suppressor gene or an oncogene, the detection of microsatellite instability in an allele in a subpopulation of cells in a biological sample is indicative of the potential for cancer or that cancer or precancer may have already developed. Further testing as described herein (either by invasive or noninvasive means) may then be conducted.

In an alternative embodiment, a "fingerprint" of Microsatellites is taken from regions associated with cancer-causing genes in a sample obtained from a patient. The fingerprint comprises the sequence of wild-type Microsatellites associated with the cancer-causing gene or genes. Once obtained, the fingerprint is stored and is used in future tests of samples from the same patient in order to monitor changes in microsatellite regions (i.e. microsatellite instability) that may be associated with the development of cancer. Changes in microsatellite length and/or sequence over time may be used to prescribe additional testing and/or treatment in order to detect and remove cancerous tissue at an early stage in its etiology.

**Claims**

1.  A method of detecting the presence of a subpopulation of cells in a biological sample obtained from an organism comprising the steps of:

    a) determining from the biological sample a number X of a first wild-type polynucleotide characteristic of a region that is not mutated, in said subpopulation of cells;
    b) determining from the biological sample a number Y of a second wild-type polynucleotide suspected of being mutated in said subpopulation of cells; and
    c) determining whether a statistically significant difference exists between said number X wherein said number X is indicative of the amount of a reference allele in said sample and said number Y wherein said number Y is indicative of the amount of wild-type target allele in said sample, the presence of a statistically significant difference being indicative of the presence of a subpopulation of cells in said biological sample.

2. A method as claimed in claim 1 wherein step a) further comprises exposing the sample to a plurality of a first oligonucleotide probe complimentary to at least a portion of nucleotide sequence of said first polynucleotide and to a plurality of a second oligonucleotide probe, complimentary to at least a portion of nucleotide sequence of said second polynucleotide under hybridization conditions, thereby to hybridize and detecting a first number of duplexes formed between said first probe and said first segment and a second number of duplexes formed between said second probe and said second segment, the number of duplexes formed between the first probe and the first polynucleotide and between the second probe and the second polynucleotide, being indicative of the number X and the number Y, and optionally wherein said sample is a mammalian tissue.

3. A method as claimed in any preceding claim for detecting allelic loss of a target allele in said subpopulation wherein said biological sample is a heterogeneous sample of cellular material wherein said plurality of a first oligonucleotide probe is a plurality of first isolation probes each capable of hybridizing only to at least a portion of either:

a coding strand of said reference allele or a compliment of a coding strand of said reference allele, and wherein said plurality of a second oligonucleotide probe is a plurality of second isolation probes each capable of hybridizing only to at least a portion of either:

a coding strand of said target allele, or a compliment of a coding strand of said target allele.

4. A method as claimed in any preceding claim for detecting a deletion in a polymorphic locus in said subpopulation of cells wherein either the number X or the number Y is indicative of the amount of maternal allele at a polymorphic locus; and wherein the other number X or Y is indicative of the amount of paternal allele at a polymorphic locus, and wherein the difference is indicative of a deletion at the polymorphic locus in said subpopulation of cells.

5. A method as claimed in any preceding claim wherein said subpopulation of cells are malignant.

6. A method as claimed in any preceding claim wherein said sample is a mammalian tissue or body fluid and wherein the presence of said statistically significant difference is indicative of the presence of a colorectal cancer or pre-cancerous lesion in said sample.

7. A method as claimed in any preceding claim wherein said biological sample is selected from the group consisting of pus, sputum, semen, urine, blood, saliva, cerebrospinal fluid, stool and biopsy tissue.

8. A method as claimed in any of claims 2-7 wherein said first oligonucleotide probe is detectably labelled with a first label, and wherein said second oligonucleotide probe is detectably labelled with a second label.

9. A method as claimed in any of claims 2-8 further comprising removing from said biological sample any unhybridized first or second oligonucleotide probe.

10. A method as claimed in any preceding claim further comprising prior to step a) the step of converting double-stranded DNA in said sample to single-stranded DNA and removing compliment to said first and second polynucleotide segments.

11. A method as claimed in claim 10 wherein the removal of said compliment comprises hybridizing said compliment to a nucleic acid probe attached to a magnetic particle and subsequently removing said magnetic particle from the sample.

12. The method according to any of claims 2 to 11 wherein said first oligonucleotides are attached to a first particle in a ratio of one first oligonucleotide probe to one particle and said second oligonucleotide probes are attached to a second particle detectably distinct from said first particle in a ratio of one second oligonucleotide probe to one second particle and wherein said detecting step comprises separating hybridized from unhybridized first and second oligonucleotide probes and subsequently passing hybridized first and second oligonucleotide probes through a detector to determine X and Y; and optionally either said first and second probe are of detectably different colours, or said first and second particles are of detectably different sizes.

13. A method as claimed in any one of claim 3-12 wherein said target allele is a tumor suppressor allele, optionally said tumor suppressor allele is a p53 allele.

**14.** A method as claimed in any one of claims 5 to 12 the polymorphic locus being identified from a database of nucleotide sequences.

**Patentansprüche**

**1.** Verfahren zur Detektion der Anwesenheit einer Subpopulation von Zellen in einer aus einem Organismus erhaltenen biologischen Probe, bei dem man:

    a) von der biologischen Probe eine Zahl X eines ersten Wildtyp-Polynukleotids bestimmt, welches in der Subpopulation von Zellen charakteristisch für eine nicht mutierte Region ist,
    b) von der biologischen Probe eine Zahl Y eines zweiten Wildtyp-Polynukleotids bestimmt, das in der Subpopulation von Zellen mutmaßlich mutiert ist, und
    c) bestimmt, ob zwischen der Zahl X, wobei die Zahl X auf die Menge eines Referenzallels in der Probe hinweist, und der Zahl Y, wobei die Zahl Y auf die Menge des Wildtyp-Zielallels in der Probe hinweist, eine statistisch signifikante Differenz existiert, wobei das Vorhandensein einer statistisch signifikanten Differenz auf die Anwesenheit einer Subpopulation von Zellen in der biologischen Probe hinweist.

**2.** Verfahren gemäß Anspruch 1, bei dem man in Schritt a) die Probe weiterhin einer Vielzahl einer ersten Oligonukleotidsonde aussetzt, die mindestens zu einem Teil der Nukleotidsequenz des ersten Polynukleotids komplementär ist, und einer Vielzahl einer zweiten Oligonukleotidsonde aussetzt, die mindestens zu einem Teil der Nukleotidsequenz des zweiten Polynukleotids unter Hybridisierungsbedingungen komplementär ist, um dadurch eine erste Anzahl von Duplexen zu hybridisieren und detektieren, die zwischen der ersten Sonde und dem ersten Segment gebildet werden, und eine zweite Anzahl von Duplexen, die zwischen der zweiten Sonde und dem zweiten Segment gebildet werden, wobei die Anzahl von Duplexen, welche zwischen der ersten Sonde und dem ersten Polynukleotid und zwischen der zweiten Sonde und dem zweiten Polynukleotid gebildet werden, auf die Zahl X und die Zahl Y hinweist, und die Probe wahlweise ein Säugergewebe ist.

**3.** Verfahren gemäß den vorangehenden Ansprüchen zur Detektion eines allelischen Verlusts eines Zielallels in der Subpopulation, worin die biologische Probe eine heterogene Probe zellulären Materials ist, worin die Vielzahl einer ersten Oligonukleotidsonde eine Vielzahl erster Isolationssonden ist, von denen jede nur an mindestens einen Teil hybridisieren kann von entweder:

    einem kodierenden Strang des Referenzallels oder einem Komplementär eines kodierenden Strangs des Referenzallels, und worin die Vielzahl einer zweiten Oligonukleotidsonde eine Vielzahl zweiter Isolationssonden ist, von denen jede nur an mindestens einen Teil hybridisieren kann von entweder:

    einem kodierenden Strang des Zielallels oder einem Komplementär eines kodierenden Strangs des Zielallels.

**4.** Verfahren gemäß den vorangehenden Ansprüchen zur Detektion einer Deletion in einem polymorphen Lokus in der Subpopulation von Zellen, worin entweder die Zahl X oder die Zahl Y auf die Menge von maternalem Allel an einem polymorphen Lokus hinweist und worin die andere Zahl X oder Y auf die Menge von paternalem Allel an einem polymorphen Lokus hinweist und worin die Differenz auf eine Deletion an dem polymorphen Lokus in der Subpopulation von Zellen hinweist.

**5.** Verfahren gemäß den vorangehenden Ansprüchen, worin die Subpopulation von Zellen bösartig ist.

**6.** Verfahren gemäß den vorangehenden Ansprüchen, worin die Probe ein Gewebe oder eine Körperflüssigkeit eines Säugers ist und worin das Vorhandensein der statistisch signifikanten Differenz auf das Vorhandensein eines kolorektalen Krebs oder einer präkanzerösen Wunde in der Probe hinweist.

**7.** Verfahren gemäß den vorangehenden Ansprüchen, worin die biologische Probe ausgewählt ist aus der Gruppe bestehend aus Eiter, Sputum, Sperma, Urin, Blut, Speichel, Cerebrospinalflüssigkeit, Stuhl und Biopsiegewebe.

**8.** Verfahren gemäß den Ansprüchen 2 - 7, worin die erste Oligonukleotidsonde mit einem ersten Kennzeichen detektierbar **gekennzeichnet** ist und worin die zweite Oligonukleotidsonde mit einem zweiten Kennzeichen detektierbar **gekennzeichnet** ist.

**9.** Verfahren gemäß den Ansprüchen 2 - 8, bei dem man von der biologischen Probe weiterhin jegliche unhybridisierte erste oder zweite Oligonukleotidsonde entfernt.

**10.** Verfahren gemäß den vorangehenden Ansprüchen, bei dem man weiterhin vor dem Schritt a) doppelsträngige DNA in der Probe in einzelsträngige DNA konvertiert und das Komplementär zu den ersten und zweiten Polynukleotidsegmenten entfernt.

**11.** Verfahren gemäß Anspruch 10, worin die Entfernung des Komplementärs die Hybridisierung des Komplementärs an eine Nukleinsäuresonde, die an ein magnetisches Partikel gebunden ist, und das anschließende Entfernen des magnetischen Partikels von der Probe umfasst.

**12.** Verfahren gemäß den Ansprüchen 2 - 11, worin die ersten Oligonukleotide im Verhältnis von einer ersten Oligonukleotidssonde zu einem Partikel an ein erstes Partikel gebunden sind und die zweiten Oligonukleotidsonden im Verhältnis von einer zweiten Oligonukleotidsonde zu einem zweiten Partikel an ein zweites Partikel gebunden sind, das detektierbar verschieden von dem ersten Partikel ist, und worin der Detektionsschritt umfasst, dass man hybridisierte von unhybridisierten ersten und zweiten Oligonukleotidsonden trennt und anschließend hybridisierte erste und zweite Oligonukleotidsonden durch einen Detektor leitet, um X und Y zu bestimmen, wobei wahlweise entweder die erste und zweite Sonde von detektierbar verschiedener Farbe sind oder die ersten und zweiten Partikel von detektierbar verschiedener Größe sind.

**13.** Verfahren gemäß den Ansprüchen 3 - 12, worin das Zielallel ein Tumorsuppressorallel ist, und wahlweise das Tumorsuppressorallel ein p53 Allel ist.

**14.** Verfahren gemäß den Ansprüchen 5 - 12, bei dem der polymorphe Lokus aus einer Datenbank von Nukleotidsequenzen identifiziert wird.

## Revendications

**1.** Procédé de détection de la présence d'une sous-population de cellules dans un échantillon biologique obtenu à partir d'un organisme comprenant les étapes consistant à :

a) déterminer à partir de l'échantillon biologique un nombre X d'un premier polynucléotide de type sauvage caractéristique d'une région qui n'est pas mutée dans ladite sous-population de cellules ;

b) déterminer à partir de l'échantillon biologique un nombre Y d'un second polynucléotide de type sauvage soupçonnée d'être muté dans ladite sous-population de cellules ; et

c) déterminer s'il existe une différence statistique entre ledit nombre X, ledit nombre X étant indicatif de la quantité d'un allèle de référence dans ledit échantillon, et ledit nombre Y, ledit nombre Y étant indicatif de la quantité de l'allèle cible de type sauvage dans ledit échantillon, l'existence d'une différence statistiquement significative étant révélatrice de la présence d'une sous-population de cellules dans ledit échantillon biologique.

**2.** Procédé selon la revendication 1 dans lequel l'étape a) comprend en outre l'exposition de l'échantillon à une pluralité d'une première sonde oligonucléotidique complémentaire d'au moins une partie de la séquence nucléotidique (ou segment) dudit premier polynucléotide et à une pluralité d'une seconde sonde oligonucléotidique, complémentaire d'au moins une partie de la séquence nucléotidique dudit second polynucléotide dans des conditions d'hybridation, de manière à hybrider et à détecter un premier nombre de duplex (ou double hélice) formés entre ladite première sonde et ledit premier segment et un second nombre de duplex formés entre ladite seconde sonde et ledit second segment, le nombre de duplex formés entre la première sonde et le premier polynucléotide et entre la seconde sonde et le second polynucléotide étant révélateur du nombre X et du nombre Y et, éventuellement dans lequel ledit échantillon est dans un tissu mammifère.

**3.** Procédé tel que revendiqué dans l'une quelconque des revendications précédentes destiné à détecter la perte allélique d'un allèle cible dans ladite sous-population où ledit échantillon biologique est un échantillon hétérogène d'un matériau cellulaire dans lequel ladite pluralité d'une première sonde oligonucléotidique est une pluralité de premières sondes d'isolement, chacune susceptible de ne s'hybrider qu'avec au moins une partie de soit:

un brin codant dudit allèle de référence, ou un complément dudit allèle de référence, et dans laquelle ladite pluralité d'une seconde sonde oligonucléotidique est une pluralité de secondes sondes d'isolement chacune capable de ne s'hybrider qu'avec au moins une partie de soit :

un brin codant dudit allèle cible, ou un complément d'un brin codant dudit allèle cible.

4. Procédé selon l'une quelconque des revendications précédentes destiné à détecter une délétion au niveau d'un locus polymorphe dans ladite sous-population de cellules dans laquelle, soit le nombre X, soit le nombre Y, est indicatif de la quantité d'allèle maternel au niveau du locus polymorphe ; et où l'autre nombre X ou Y est indicatif de la quantité d'allèle paternel au niveau d'un site polymorphe, et où la différence est révélatrice d'une détection au niveau du site polymorphe dans ladite sous-population cellulaire.

5. Procédé selon l'une quelconque des revendications précédentes dans lequel ladite sous-population de cellules est maligne.

6. Procédé selon l'une quelconque des revendications précédentes dans lequel ledit échantillon est un tissu ou un fluide corporel et dans lequel la présence de ladite différence statistiquement significative est révélatrice de la présence d'un cancer colorectal ou d'une lésion précancéreuse dans ledit échantillon.

7. Procédé selon l'une quelconque des revendications précédentes dans lequel ledit échantillon biologique est choisi parmi le groupe constitué par le pus, les expectorations, le sperme, l'urine, le sang, la salive, le liquide céphalorachidien, les selles et une biopsie tissulaire.

8. Procédé selon l'un quelconque des revendications 2 à 7 dans lequel ladite première sonde oligonucléotidique est marquée de manière détectable avec un premier marqueur, et dans lequel la seconde sonde oligonucléotidique est marquée de manière détectable avec un second marqueur.

9. Procédé selon l'une quelconque des revendications 2 à 8 comprenant en outre l'élimination dudit échantillon biologique de toute première ou seconde sonde oligonucléotidique non hybridée.

10. Procédé selon l'une quelconque des revendications précédentes comprenant en outre avant l'étape a) une étape de conversion de l'ADN double brin présent dans ledit échantillon en ADN simple brin et l'élimination du complément desdits segments du premier et du second polynucléotide.

11. Procédé selon la revendication 10 dans lequel l'élimination dudit complément comprend l'hybridation dudit complément à une sonde d'acide nucléique liée à une particule magnétique puis l'élimination ultérieure de ladite particule magnétique de l'échantillon.

12. Procédé selon l'une quelconque des revendications 2 à 11 dans lequel lesdites premières sondes oligonucléotidiques sont liées à une première particule dans un rapport d'une première sonde oligonucléotidique pour une particule et lesdites secondes sondes oligonucléotidiques sont liées à une seconde particule distincte de manière détectable de ladite première particule dans un rapport d'une seconde sonde oligonucléotidique pour une seconde particule et où ladite étape de détection comprend la séparation des premières et secondes sondes oligonucléotidiques hybridées à partir des sondes non hybridées puis le passage ultérieur des première et seconde sondes oligonucléotidiques hybridées sur un détecteur afin de déterminer X et Y ; et éventuellement lesdites premières et secondes sondes sont de couleurs différentes de manière détectable ou lesdites première et seconde particules sont de tailles différentes de manière détectable.

13. Procédé selon l'une quelconque des revendications 3 à 12 dans lequel ledit allèle cible est un allèle suppresseur de tumeur, ledit allèle suppresseur de tumeur étant éventuellement l'allèle p53.

14. Procédé selon l'une quelconque des revendications 5 à 12, dans lequel le locus polymorphe est identifié à partir d'une base de données de séquences nucléotidiques.

FIG. 1

FIG. 2

1:                    3'ACGCTACGG5'

2:     5'....ATCGGCTTACTGCGATGCC....3'

M

3:     3'....TAGCCGAATGACGCATCGG....5'

4:     5'ATCGGCTTA3'

1:                    3'ACGCTACGG5'

2:     5'....ATCGGCTTATTGCGATGCC....3'

F

3:     3'....TAGCCGAATAACGCTCGG....5'

4:     5'ATCGGCTTA3'

# FIG. 3

N

A

FIG. 4A

N

B

FIG. 4B

FIG. 5